(19) Europäisches Patentamt / European Patent Office / Office européen des brevets

(11) **EP 3 253 201 B1**

(12) **EUROPEAN PATENT SPECIFICATION**

(45) Date of publication and mention of the grant of the patent:
**22.07.2020 Bulletin 2020/30**

(51) Int Cl.:
*A61P 33/00* (2006.01)   *A01N 25/02* (2006.01)
*A01N 25/10* (2006.01)   *A01N 47/34* (2006.01)

(21) Application number: **16702440.5**

(22) Date of filing: **02.02.2016**

(86) International application number:
**PCT/EP2016/052204**

(87) International publication number:
**WO 2016/124610 (11.08.2016 Gazette 2016/32)**

(54) **A FORMULATION FOR TREATMENT OF BLOWFLY STRIKE**

FORMULIERUNG ZUR BEHANDLUNG VON SCHMEISSFLIEGENBEFALL

FORMULATION POUR LE TRAITEMENT D'UNE PIQÛRE DE MYIASE

(84) Designated Contracting States:
**AL AT BE BG CH CY CZ DE DK EE ES FI FR GR HR HU IE IS IT LI LT LU LV MC MK MT NL NO PL PT RO RS SE SI SK SM TR**

(30) Priority: **03.02.2015 EP 15153652**

(43) Date of publication of application:
**13.12.2017 Bulletin 2017/50**

(73) Proprietor: **Bimeda Finance S.A.R.L.**
**1331 Luxembourg (LU)**

(72) Inventors:
• **MOLINS ALBANELL, Francisco Javier**
**County Dublin (IE)**

• **SMITH, Brendan Gerard**
**Dublin**
**D03V9F7 (IE)**
• **GUPTA, Sandeep**
**Blessington**
**County Wicklow (IE)**

(74) Representative: **John A. O'Brien & Associates**
**Shannon Lodge**
**Casement Road**
**Bandon**
**County Cork, P72 TN24 (IE)**

(56) References cited:
**EP-A2- 0 306 055       WO-A1-2011/120427**
**WO-A1-2011/157101    WO-A1-2011/157102**
**US-A1- 2013 065 846**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Printed by Jouve, 75001 PARIS (FR)

**Description**

Introduction

The invention relates to a formulation to protect against blowfly (*Lucilia* spp.) in sheep.

Blowfly strike is the popular name for cutaneous myiases. Caused by the Blowfly (Lucilia), cutaneous myiasis are skin infections with fly maggots that feed on the superficial tissues of their victims. They are particularly abundant and damaging for the sheep industry in many regions of the world with temperate climates including Australia, New Zealand, Great Britain, Ireland and South Africa.

Prevalence and incidence depend strongly on climatic and ecologic conditions. In the UK it is estimated that up to 80% of sheep farms are affected each year. As a general rule sheep blowflies prefer moderate and warm climates. In most endemic regions blowfly strike is a summer pest with peaks during the hottest months. Rainy weather strongly favours blowfly strike.

Blowfly strike is a serious pest for sheep. Ewes and lambs are especially affected. The maggots virtually eat their host alive, which is highly stressing and annoying for the animals. Secondary bacteria infect the wounds and the hosts' organism. The maggots produce also ammonia, which is poisonous for sheep. As a consequence affected sheep are depressed, stop feeding, suffer from fever, inflammations, blood loss, etc. Left untreated blowfly strike is often fatal for affected sheep, especially for lambs.

Given the potential for economic loss and suffering, sheep farmers in high risk areas will typically administer a preventative dose of an ectoparasiticide before the onset of the season. Typically the ectoparasiticide is applied by either dipping the sheep in a bath in which the active ingredient is dissolved or by pouring / spraying directly on to the sheep's back.

Commonly used chemicals include organophosphates (such as diazinon), syntethic pyrethroids (such as cypermethrin), triazines (such as cyromazine) and pyrimidines (such as dicyclanil).

All of these chemicals provide varying levels of protection from fly strike (from 6 to 16 weeks) and also have varying meat withdrawal periods (the time that must lapse between treatment and when it is safe to slaughter the animal for human consumption). Typically with conventional treatments it is found that the longer the period of protection, the longer the period of meat withdrawal.

WO2011/120427A describes a pour-on formulation comprising a $C_2$-$C_4$ dialkylene glycol mono/di-Cl-$C_4$ alkyl ether as a carrier.

Statements of Invention

According to the invention, Novaluron for use in the prophylaxis of blow fly strike in sheep is provided.

Novaluron may be present in the formulation at a concentration of from 2% to 4% by weight. Novaluron may be present in the formulation at a concentration of about 3% by weight.

The butyl dioxotol in one case comprises from 90 to 95% w/w of the formulation.

Polyvinylpyrollidone may comprise from 3 to 6% w/w of the formulation. In one case the PVP has a molecular weight of 5 to 500 kDa. The PVP may have a molecular weight of from 40 to 80 kDa.

In one embodiment the carrier comprises N-methyl pyrollidone.

We have found that Novaluron gives a long period of protection against blowfly, while requiring an extremely low meat withdrawal period (maximum 1 or 2 days).

Detailed Description

Novaluron is a known insect growth regulator (IRG) available from Makhteshim Chemicals Works Limited. Novaluron is described in US5,089,525.

Butyl dioxotol is available from Ineos, France, is present in the formulation of the invention in an amount of from 90% to 95% w/w. Butyl dioxotol is also known as diethylene glycol butyl ether, which acts as a solvent in the formulation. Butyl ether of diethylene glycol is a glycol ether.

N-Methylpryrollidone is available from Nanjing Jinlong Chemical Company Ltd, China.

Polyvinylpyrollidone (PVP) or povidone is available in a range of molecular weights. In the pour-on formulations below Povidone K-30 available from BASF, Germany is used which has a molecular weight in the range 40kDa to 80kDa. The PVP is present in the pour-on formulation of the invention in an amount of from 3% to 6% w/w and acts within the formulation to assist with persistence on the sheep's fleece.

Eurocert Green is a dye which is present in the formulation to provide a temporary identification of treated sheep. It is available from Sensient Colours Ltd, UK, CAS No. 3087-16-9 (Methanaminium,N-[4-[[4-(dimethylamino)phenyl](2-hydroxy-3,6-disulfo-1-naphthalenyl)methylene]-2,5-cyclohexadien-1-ylidene]-N-methyl-,inner salt, sodium salt (1:1))

## Example

Six pour-on formulations were prepared which comprised two different matrices of excipients (Formulation A and Formulation B) into which 2%, 3% and 4% of Novaluron was dissolved.

### Formulation A: 2% Novaluron Batch Number: RD-SB1211-03

| Material | Code | Batch Number | Batch Requirement | Actual Values |
|---|---|---|---|---|
| Butyl dioxitol | 6 BUT 001 | R1221556 | QS. To IL | Approx. 840ml |
| N-methylpyrrolidone | 6 NMP001A | R1221334 | 150g | 150.13g |
| Novaluron Technical | Not Applicable | 91861079 | 20g | 20.28g |
| Eurocert Green | 6EUR002 | R1120596 | 0.2g | 0.21g |
| 1L Back Pack Flat Bottom (HDPE) | 8 CON 113 | Not Applicable | 1 | 1 |
| 38mm Standard Cap | 8 cap 001 | Not Applicable | 1 | 1 |

- Novaluron was dissolved in NMP with stirring. Dissolution time <2minutes

- Eurocert green added and mixed for five minutes.

- QS to 1L with Butyl dioxitol. Approximately 840ml required

- Mixed for ten minutes to produce a homogenous solution.

- Appearance: A clear blue solution

- 50ml of solution was filled into a clear vial and stored at ambient conditions

- 1 Litre Back Pack Flat Bottom HDPE bottle filled, and sealed with 38mm Standard Cap.

### Formulation A: 3% Novaluron Batch Number: RD-SB1211-04

| Material | Code | Batch Number | Batch Requirement | Actual Values |
|---|---|---|---|---|
| Butyl dioxitol | 6 BUT 001 | R1221556 | QS. To IL | Approx. 820ml |
| N-methylpyrrolidone | 6 NMP001A | R1221334 | 150g | 150.42g |
| Novaluron Technical | Not Applicable | 91861079 | 30g | 30.01g |
| Eurocert Green | 6EUR002 | R1120596 | 0.2g | 0.22g |
| 1L Back Pack Flat Bottom (HDPE) | 8 CON 113 | Not Applicable | 1 | 1 |
| 38mm Standard Cap | 8 cap 001 | Not Applicable | 1 | 1 |

- Novaluron was dissolved in NMP with stirring. Dissolution time <2minutes

- Eurocert green added and mixed for five minutes.

- QS to 1L with Butyl dioxitol. Approximately 820ml required

- Mixed for ten minutes to produce a homogenous solution.

- Appearance: A clear blue solution

- 50ml of solution was filled into a clear vial and stored at ambient conditions

- 1 Litre Back Pack Flat Bottom HDPE bottle filled, and sealed with 38mm Standard Cap.

**Formulation A: 4% Novaluron Batch Number: RD-SB1211-05**

| Material | Code | Batch Number | Batch Requirement | Actual Values |
|---|---|---|---|---|
| Butyl dioxitol | 6 BUT 001 | R1221556 | QS. To IL | Approx. 800ml |
| N-methylpyrrolidone | 6 NMP001A | R1221334 | 150g | 149.82g |
| Novaluron Technical | Not Applicable | 91861079 | 40g | 40.16g |
| Eurocert Green | 6EUR002 | R1120596 | 0.2g | 0.19g |
| 1L Back Pack Flat Bottom (HDPE) | 8 CON 113 | Not Applicable | 1 | 1 |
| 38mm Standard Cap | 8 cap 001 | Not Applicable | 1 | 1 |

- Novaluron was dissolved in NMP with stirring. Dissolution time <2minutes

- Eurocert green added and mixed for five minutes.

- QS to 1L with Butyl dioxitol. Approximately 800ml required

- Mixed for ten minutes to produce a homogenous solution.

- Appearance: A clear blue solution

- Three 50ml glass vials were filled with this solution, and stored at refridgerated, ambient and accelerated conditions.

- 1 Litre Back Pack Flat Bottom HDPE bottle filled, and sealed with 38mm Standard Cap.

**Formulation B: 2% Novaluron Batch Number: RD-SB1211-06**

| Material | Code | Batch Number | Batch Requirement | Actual Values |
|---|---|---|---|---|
| Butyl dioxitol | 6 BUT 001 | R1221556 | QS. To IL | Approx. 950ml |
| Povidone K30 | 6 POV 001 | R1221243 | 50g | 49.92g |
| Novaluron Technical | Not Applicable | 91861079 | 20g | 19.93g |
| Eurocert Green | 6EUR002 | R1120596 | 0.2g | 0.23g |
| 1L Back Pack Flat Bottom (HDPE) | 8 CON 113 | Not Applicable | 1 | 1 |
| 38mm Standard Cap | 8 cap 001 | Not Applicable | 1 | 1 |

- Povidone slowly added to 500g of Butyl Dioxitol with stirring. Dissolution time 10minutes @1500RPM

- Novaluron dissolved in this solution, with stirring. Dissolution time < 5minutes

- Eurocert green added and mixed for five minutes.

- QS to 1L with Butyl dioxitol. Approximately 450ml was required

- Mixed for ten minutes to produce a homogenous solution.

- Appearance: A clear blue solution

- 50ml of solution was filled into a clear vial and stored at ambient conditions

- 1 Litre Back Pack Flat Bottom HDPE bottle filled, and sealed with 38mm Standard Cap.

**Formulation B: 3% Novaluron Batch Number: RD-SB1211-07**

| Material | Code | Batch Number | Batch Requirement | Actual Values |
|---|---|---|---|---|
| Butyl dioxitol | 6 BUT 001 | R1221556 | QS. To IL | Approx. 940ml |
| Povidone K30 | 6 POV 001 | R1221243 | 50g | 50.51g |
| Novaluron Technical | Not Applicable | 91861079 | 30g | 30.17g |
| Eurocert Green | 6EUR002 | R1120596 | 0.2g | 0.20g |
| 1L Back Pack Flat Bottom (HDPE) | 8 CON 113 | Not Applicable | 1 | 1 |
| 38mm Standard Cap | 8 cap 001 | Not Applicable | 1 | 1 |

- Povidone slowly added to 500g of Butyl Dioxitol with stirring. Dissolution time 10minutes @1500RPM

- Novaluron dissolved in this solution, with stirring. Dissolution time < 5minutes

- Eurocert green added and mixed for five minutes.

- QS to 1L with Butyl dioxitol. Approximately 440ml was required

- Mixed for ten minutes to produce a homogenous solution.

- Appearance: A clear blue solution

- 50ml of solution was filled into a clear vial and stored at ambient conditions

- 1 Litre Back Pack Flat Bottom HDPE bottle filled, and sealed with 38mm Standard Cap.

**Formulation B: 4% Novaluron Batch Number: RD-SB1211-08**

| Material | Code | Batch Number | Batch Requirement | Actual Values |
|---|---|---|---|---|
| Butyl dioxitol | 6 BUT 001 | R1221556 | QS. To IL | Approx. 930ml |
| Povidone K30 | 6 POV 001 | R1221243 | 50g | 49.72g |
| Novaluron Technical | Not Applicable | 91861079 | 40g | 40.31g |
| Eurocert Green | 6EUR002 | R1120596 | 0.2g | 0.20g |
| 1L Back Pack Flat Bottom (HDPE) | 8 CON 113 | Not Applicable | 1 | 1 |
| 38mm Standard Cap | 8 cap 001 | Not Applicable | 1 | 1 |

- Povidone slowly added to 500g of Butyl Dioxitol with stirring. Dissolution time 10minutes @1500RPM

- Novaluron dissolved in this solution, with stirring. Dissolution time < 5minutes
- Eurocert green added and mixed for five minutes.
- QS to 1L with Butyl dioxitol. Approximately 430ml was required
- Mixed for ten minutes to produce a homogenous solution.
- Appearance: A clear blue solution
- Three 50ml glass vials were filled with this solution, and stored at refrigerated, ambient and accelerated conditions.
- 1 Litre Back Pack Flat Bottom HDPE bottle filled, and sealed with 38mm Standard Cap.

Six groups, each of eleven sheep had a different formulation applied to them and larval implant challenges were undertaken on each formulation at eight time-points, ranging from fourteen days post treatment to one hundred and five days post application.

It was found that Formulation B, with a 3% inclusion of Novaluron gave excellent cover and was still providing cover up to fifteen weeks post application. It is possible that NMP in formulation A interfered with persistence on the fleece.

Further studies, designed to assess the levels of Novaluron in tissues and fleece were conducted. This study showed that the residues of Formulation B 3%, were below what is expected to be established as the Maximum Residues Limit at two days post treatment.

The formulation is effective in prevention and treatment of ectoparasites on sheep and goats with a low meat withdrawal period (maximum 1 or 2 days).

In more detail, a study was carried out to evaluate the dose levels and persistent efficacy of the pour-on formulation to protect against blowfly (*Lucilia* spp.) in sheep following a routine application of the investigational veterinary product (IVP). This was carried out by assessing the effects on artificial implantations treatment. The secondary objective of this study was to provide samples to determine the depletion of the IVP in the target tissues (kidney, liver, muscle and fat) of sheep following a routine application of the IVP. This was carried out by collecting tissue samples at one, two and three weeks post IVP administration.

The study consisted of seven treatment groups, consisting of 69 sheep which received IVP by topical application on day 0 or remained untreated, as per Table 1 below. Animals arrived at Location 1, 15 days prior to day 0 for acclimatisation on the grazing areas that were used to accommodate the animals for study. Animals were necropsied for tissue residue analysis on three occasions (total of six per treatment group, excluding negative controls), or challenged for assessment of does rates and persistent efficacy on seven occasions (total of five animals per treatment group T2 - T7, and three animals T1) as per Table 1 below. The experimental unit was the individual animal. All parameters were assessed for each individual animal. Staff were not blinded to treatment to ensure correct bio-security to prevent cross-contamination.

**Table 1: Study Design**

| Treatment Group | Treatment Material | Study Day | Dose Rate | Tissue Collection Days (Necropsies)* | Challenge Days** | Starting No Animals |
|---|---|---|---|---|---|---|
| 1 | None | N/A | N/A | N/A | 14, 21, 42, 58, 70, 84, 98, 105 | 3 |
| 2 | Formulation A | 0 | 2% | 7, 14, 21 | 14, 21, 42, 58, 70, 84, 98, 105 | 11 |
| 3 | Formulation A | 0 | 3% | 7, 14, 21 | 14, 21, 42, 58, 70, 84, 98, 105 | 11 |
| 4 | Formulation A | 0 | 4% | 7, 14, 21 | 14, 21, 42, 58, 70, 84, 98, 105 | 11 |
| 5 | Formulation B | 0 | 2% | 7, 14, 21 | 14, 21, 42, 58, 70, 84, 98, 105 | 11 |
| 6 | Formulation B | 0 | 3% | 7, 14, 21 | 14, 21, 42, 58, 70, 84, 98, 105 | 11 |
| 7 | Formulation B | 0 | 4% | 7, 14, 21 | 14, 21, 42, 58, 70, 84, 98, 105 | 11 |
| * Two animals per occasion from T2, T3, T4, T5, T6 & T7 | | | | | | |
| ** Five animals per occasion from T2, T3, T4, T5, T6 & T7 and three from T1 | | | | | | |

## Allocation to Treatment

Animals were allocated to treatment groups using live-weight, condition score and fleece length/condition as the primary variables, 15 days prior to day 0 (see Appendix 3). Two animals were swapped on the treatment allocation 14 days prior to day 0 to move one animal from T1 to the necropsy group in T2, this was a result of the animal's behaviour making it unlikely to be retained on study for the duration of the study. There was no significant difference in live-weight between the treatment groups at allocation to treatment 14 days prior to day 0 (p = 0.995). Animals were randomly selected for necropsy on 16 November 2012, 18 days prior to day 7.

Dosage form:             Novaluron pour-on

Formulations:            A & B at 2, 3 and 4% Novaluron

Dose Rate:               10-15kg animals = 15ml
                         16-20kg animals = 20ml
                         21-30kg animals = 25ml
                         31-50kg animals = 30ml
                         > 50kg = 40ml

Administration method:   Topical pour-on along back using recommended applicator gun. Applied by fan spray, 50% down back and 50% across crutch

| Formula | Concentration | Batch Number |
|---------|--------------|--------------|
| Formulation A | 2% | RD-SB1211-03 |
| Formulation A | 3% | RD-SB1211-04 |
| Formulation A | 4% | RD-SB1211-05 |
| Formulation B | 2% | RD-SB1211-06 |
| Formulation B | 3% | RD-SB1211-07 |
| Formulation B | 4% | RD-SB1211-08 |

## Animals Details

Species:    Ovine
Breed:      Lleyn crosses
Age:        1 to 5 years
Sex:        Female
Origin:     Commercial Farms UK
Owner:      ADAS UK Ltd

Identification: Individual statutory and colour coded management tags

Sheep were selected from a group of approximately 120 animals. The selected sheep:

- were in good physical condition
- had medium/long fleece (greater than approximately 4cm) and the fleece was in good condition
- were not treated with an ectoparasiticide in the previous six months
- were not treated with an avermectin in the previous two months

Sheep were grazed in separate paddocks per treatment group for 44% of the study period from day 0. Sheep were group housed per treatment group during challenge procedures and implant checks. They were also housed on

EP 3 253 201 B1

occasions when weather conditions made it unsuitable for the grazing paddocks to be used.

### IVP Preparation and Administration

IVP was administered as a single topical treatment per animal according to individual liveweight on day 0 (see section 8.1) using the recommended applicator gun. Each animal was held securely. IVP was applied as a fan-spray to the surface of the fleece on the back and hindquarters of the sheep using a pour-on gun fitted with a fan-spray nozzle. Half the dose was applied as a line covering the shoulders, back and flanks, and half in an arch over the rump. The distance between the nozzle and the fleece was approximately 20cm. Application was made to healthy skin avoiding any scabs, skin lesions or extraneous foreign matter.

The applicator gun was tested and calibrated using water prior to use on day 0. A separate applicator gun was used for each formulation and concentration applied.

### Challenge Procedures

Five animals per treatment groups T2, T3, T4, T5, T6 and T7 and three animals from T1 were challenged with *Lucilia sericata* (blowfly) L1 on days 14, 21, 42, 58, 70, 84, 98 and 105 post treatment.

Animals were moved indoors for challenge and remained housed for at least three days while checks of the larval implants are carried out. On a number of occasions animals were housed earlier/longer than planned as a result of weather conditions making the paddocks unsuitable for use (see Appendix 2 for further details of housing/grazing).

Approximately 50-100 L1 were implanted into each of two sites per animal on each challenge occasion. One site was 10cm and the other 20cm from the midline of the back. Implants initially started over the shoulder of the animal, working backward throughout the course of the study.

On challenge days 14 and 21, larval 'plugs' were prepared at location 2. Between 50-100 L1 were placed inside of a piece of cotton will and moistened with liver fluids, these were then covered over to contain the larvae and transported in a secure container at ambient temperature directly to Location 1. From challenge day 42 onwards, newly emerged larvae were collected directly from Location 2 on the morning of implant and transported on raw liver in a secure container at ambient temperature to Location 1.

Up to day 42 challenge, the pre-loaded larval plugs were removed from the container and placed on abraded skin (about 1cm squared), the plug was pulled open a bit to expose the larvae to the skin and held in place using fleece from around the site and an elastic band.

Post day 42 challenge, larvae on liver were kept in polystyrene boxes containing heat packs to protect them from the elements and 50-100 L1 were removed directly from the liver, for each implant site, and placed directly onto abraded skin. They were then covered with a small moist cotton wool plug (with lukewarm distilled water), and held in place using the fleece around the site and an elastic band.

Skin was abraded by carefully scraping fleece from the top layer of the skin using a scalpel blade. Staff carrying out implanting of larvae changed gloved between each animal.

### Challenge Assessments

Implants and surrounding areas were checked at approximately 24, 48 and 72 hours post challenge.

The presence of live or dead larvae and progression of larvae from L1, L2 and L3 was recorded. An estimate of numbers of live larvae present was made and recorded as follows:

    0 = none alive
    1 = up to 10 alive
    2 = > 10 and < 30 alive
    3 = > 30 and < 50 alive
    4 = > 50 alive

If no larvae were seen on the site, this was documented as either 'n/a' being written in all boxes (challenge assessment up to day 42) or by 'none seen' being written (day 42 onwards).

In addition any malformation or strange appearance of the larvae was scored and recorded as follows:

    A = normal active larvae
    B = some slowing of movement or larvae under-sized
    C = marked slower activity and/or malformations
    D = severely affected, almost dead

Along with numbers and appearance, from day 21, the location of larvae was recorded as being found on either the 'skin', 'plug' or 'skin' and 'plug' ('plug' referring to cotton wool, pre-loaded or otherwise).

Any L3 larvae present on any animals at any assessment point were removed. Larval plugs were discarded following larval progression to L3 at any check point. Once checks has been completed at 72 hours post challenge, all remaining larvae (L1, L2 or L3) were removed by hand from the animals and all plugs discarded.

On one occasion (day 59, sheep 021, treatment 6), early signs of strike resulted in L2 larvae being removed at 24 hours as well as the L3 present in order to prevent fill strike occurring on sit B for welfare of the animal. The plug was not replaced in this instance.

## Fleece Samples

On days 44 and 84 (six and 12 weeks post treatment), two samples of fleece were taken from all animals (Samples A and B), to be analysed for the levels of active ingredient still present.

Fleece samples were taken using stainless steel, curved scissors. Cutting the fleece as close to the skin as possible - approximately 0.5 g of wool was taken from each of four sample sites. The samples were pooled for each animal. Sample sites were two at 10cm from the midline of the back, and two at 20cm from the middling of the back, approximately halfway between the base of the neck and the top of the tail.

All samples were kept frozen at $\leq$ - 15°C until transfer of Sample A, for each animal and occasion, the Location 8 for analysis on 25 February 2013, using frozen transport at approximately -20°C. Sample transport temperatures were monitored using a data logger throughout transport and the temperature was below -15°C throughout. Sample B, for each animal and occasion, will remain frozen at ADAS Drayton for a maximum of one year after receipt by Location 8, unless required for analysis by Location 8.

## Bio-security

Animals were grazed in separate paddocks per treatment group. Each paddock was fenced with electric fencing preventing access to hedged and preventing contact between groups. On study day 17, treatments 5 and 7 were returned to the incorrect paddocks (paddocks 5 and 7 respectively) following a period of indoor housing for challenge purposes (see Deviation 3, section 15.2). On 8 January 2013 when bringing the sheep for housing from grazing, sheep 006 and 009 from treatment 3 jumped the fence into paddock 7 (treatment 5). They were in paddock 7 for a maximum of 5 minutes before being removed. Each treatment group was assigned to a dedicated room in G3 or G4 for challenge procedures and assessments for the duration of the study. See appendix 2 for details of grazing and housing.

Separate PPE (overalls and gloves) were used for each treatment group. Wellington boots were washed off between treatment groups.

Study activities were performed by the same personnel, and as per the order below, except on two occasions, one, as mentioned above, when the treatment 5 and 7 were returned to the incorrect paddocks (see Deviation 3), and once at the end of the study when it was agreed to change the order in which treatments were challenged to pre-empt a potential lack of larvae with which to implant the animals (see Amendment 9).

| Treatment Group | Paddock | Room |
|---|---|---|
| T1 (White) | 1 | G4-5 |
| T2 (Red) | 2 | G4-4 |
| T3 (Yellow) | 3 | G4-3 |
| T4 (Blue) | 4 | G4-2 |
| T5 (Orange) | 7 | G3-1 |
| T6 (Green) | 6 | G3-2 |
| T7 (Pink) | 5 | G3-3 |

## Necropsy, Blood and Tissue Sampling for Residues

### Euthanasia

On days 7, 14 and 21, 12 animals were humanely euthanased using a schedule 1 method and necropsies conducted.

Euthanasia was by means of captive bolt stunning followed by use of pithing rod and exsanguination, performed by appropriately trained personnel.

On each necropsy day, two treated animals from T2, T3, T4, T5, T6 and T7 were euthanased. Animals were randomly selected for necropsy by the investigator (see section 7.2).

**Necropsy**

On days 7, 14 and 21 samples of loin muscle, sub-cutaneous fat, omental fat, liver (equal amounts of each lobe) and kidneys were collected from each sheep immediately after termination.

During necropsy, extreme care was taken to avoid any contact of the fleece with sub-cutaneous fat, skeletal muscle and internal viscera. This was achieved by careful removal of the skin with the fleece attached before the collection of any samples began.

Two replicate samples (R1 and R2) were collected for each specimen type (loin muscle, sub-cutaneous fat, omental fat, liver and kidneys).

For muscle and liver samples at least 446g of each specimen was collected. Staff ensured that R1 contained at least 200g. Total samples were divided approximately equally between R1 and R2. The sample collected from the lover was composed of at least three sub-samples from the organ.

For sub-cutaneous fat and omental fat, the R1 sample contained at least 61g (or maximum available) and kidneys were cut in half and half o the left kidney and half of the right kidney made up each replicate.

All replicates were uniquely labelled and double-wrapped in plastic bags, with an additional label between the bags. They were then transferred to a freezer set to maintain a temperature of ≤ -15°C.

**Blood Sampling**

Blood samples (50ml per animal except animals 063 and 034 on day 14, where only just over 10ml and 40ml were collected respectively, see Deviation 2) were collected from each animal necropsied on days 7, 14 and 21. Samples were sent for analysis at Location 8 on 25 February 2013.

Blood samples were collected by venepuncture into a maximum of five green top vaccutainer tubes (10ml in each tube).

To prevent deterioration, samples were processed as soon as possible after collection. Samples were collected in cold conditions (average temperature on day 7 was 2.67°C, on day 14 was - 2.79°C and on day 21 was 5.54°C) which prevented any deterioration prior to processing. The samples were centrifuged at 3000 rpm. Plasma was aliquoted into a maximum of 6 aliquots until all plasma had been retrieved from the blood samples.

**Sample Storage and Transfer**

Necropsy samples (including plasma samples) were stored in a freezer set to maintain a temperature of ≤ -15°C. All R1 samples and three aliquots of plasma per animal (aliquots A, B and C) were transferred frozen to Location 8 for analysis of residues on 25 February 2013 using frozen transport at approximately -20°C. Sample transport temperatures were monitored using a data logger throughout transport and the temperature was below -15°C throughout. The R2 sample will remain frozen at ADAS for a maximum of one year after receipt by Location 8, unless required for analysis by Location 8.

**Data Analysis**

Data from the treated groups (T2 - T7) was compared with the control group (T1) using the appropriate statistical tests as outlined below for each data set and accepting a level of probability of less than or equal 0.05 as indicating significance. However, the actual P value is reported where applicable. Statistical analysis was carried out by the ADAS Statistician using GenStat 12.1.

Data sets showing the presence of L3 or L2 were analysed using logistic regression analysis. The data was analysed within Genstat regression, where the data was transformed using a log transformation and the analysis carried out on the data. The means were then back transformed to provide the proportion of animals with L3 per treatment group. The initial analysis was carried out using just the individual treatments, following this, data was analysed using a factorial structure of the two formulations times the three doses.

The data sets for larval stages present on skin at 10cm site, at 20cm site and at 10 and 20cm combined; on plug at 10cm site, at 20cm site and at 10 and 20cm combined; on skin and plug at 10cm site, at 20cm site and at 10 and 20cm combined, were analysed using a logistic regression analysis (as per presence of L3 and L2 above), using the seven treatment groups. No factorial analysis was carried out. In addition the proportions of animals in each treatment group that reached L3 were analysed and distribution tables were created for each time point, to show the numbers of each animal in each treatment reaching each larval stage.

Data sets of larval numbers present were analysed using the seven individual treatments, following this, data was analysed using a factorial structure of the two formulations times the three doses. Data was analysed by analysis of variance (ANOVA) and least significant difference tests.

Percentage effectiveness was calculated comparing numbers of live larvae (L1, L23 and L3) for controls against each treatment group at 48 and 72 hours post challenge. In addition percentage effectiveness was calculated assessing the progression to L3 at any stage post challenge.

Percent effectiveness was calculated as follows:

$$effectiveness(\%) = \frac{(GM\ of\ the\ number\ of\ parasites\ in\ control\ animals) - (GM\ of\ the\ number\ of\ parasites\ in\ treated\ animals)}{(GM\ of\ the\ number\ if\ parasites\ in\ control\ animals)}\ x\ 100$$

The geometric mean cannot be calculated if some numbers are equal to zero, therefore a transformation of all values was performed for all calculations of geometric mean by increasing the raw numbers by 1.

## Control

All control animals (T1) and five animals per treatment groups (T2 - T7) were challenged with *Lucilia sericata* (blowfly) L1 for assessment of dose rates and persistent efficacy on seven occasions on study days 14, 28, 42, 56, 70, 84, 105. There was one failed implant challenge on day 98 that was not used to evaluate persistent efficacy. Animals were moved indoors for challenge and remained housed for at least three days while checks of the larval implants were carried out. Approximately 50 - 100 L1 were implanted onto each of two sites per animal on each challenge occasion. One site was 10cm and the other 20cm from the midline of the back. Implants and surrounding areas were checked at approximately 24, 48 and 72 hours post challenge. The presence of live of dead larvae and progression of larvae from L1 to L2 or L3 was recorded and an estimate of numbers of live larvae present was made and recorded.

**Treatment with T2** resulted in greater than 90% effectiveness on one occasion (post challenge on day 70). There was no effect of treatment at any point on numbers of animals with live larvae present at 24 hours post challenge for any challenge occasion. There were significantly fewer animals with live larvae at 48 and 72 hours post challenge and L3 only at any point post challenge, for challenges on day 14 and day 70 and for L3 only on day 84. There were significantly lower numbers of live larvae at 24, 48 and 72 hours post challenge and L3 only at 72 hours post challenge for challenges on days 14, 70, 84 and 105.

**Treatment with T3** resulted in greater than 90% effectiveness on four occasions (post challenge on days 14, 42, 40 and 84). There was no effect of treatment at any point on numbers of animals with live larvae present at 24 hours post challenge for any challenge occasion. There were significantly fewer animals with live larvae at 48 and 72 hours post challenge and L3 only at any point post challenge, for challenges on days 14, 42, 70 and 84. There were significantly lower numbers of live larvae at 24, 48 and 72 hours post challenge and L3 only at 72 hours post challenge for challenges on days 14, 70, 84 and 105.

**Treatment with T4** resulted in greater than 90% effectiveness on four occasions (post challenge on days 14, 42, 70 and 84). There was no effect of treatment at any point on numbers of animals with live larvae present at 24 hours post challenge for any challenge occasion. There were significantly fewer animals with live larvae at 48 and 72 hours post challenge and L3 only at any point post challenge, for challenges on days 14, 42, 70, 84 and 105. There were significantly lower numbers of live larvae at 24, 48 and 72 hours post challenge and L3 only at 72 hours post challenge for challenges on days 14, 42 (at 72 hours post challenge only), 70, 84 and 105.

**Treatment with T5** resulted in greater than 90% effectiveness on five occasions (post challenge on days 14, 42, 70, 84 and 105). There was no effect of treatment at any point on numbers of animals with live larvae present at 24 hours post challenge for any challenge occasion. There were significantly fewer animals with live larvae at 48 and 72 hours post challenge and L3 only at any point post challenge, for challenges on days 14, 42, 70, 84 and 105. There were significantly lower numbers of live larvae at 24, 48 and 72 hours post challenge and L3 only at 72 hours post challenge for challenges on days 14, 42, 70, 84 and 105 (at 48 and 72 hours post challenge only).

**Treatment with T6** resulted in greater than 90% effectiveness on four occasions (post challenge on days 42, 70, 84 and 105). There was no effect of treatment at any point on numbers of animals with live larvae present at 24 hours post challenge for any challenge occasion. There were significantly fewer animals with live larvae at 48 and 72 hours post challenge and L3 only at any point post challenge, for challenges on days 42, 70, 84 and 105. There were significantly lower numbers of live larvae at 24, 48 and 72 hours post challenge for challenges on days 21 (24 hours post challenge only), 42 (48 and 72 hours post challenge only), 70, 84 and 105. There were significantly lower numbers of L3 present at 72 hours post challenge compare to the controls following challenge on days 70, 84 and 105.

**Treatment with T7** resulted in greater than 90% effectiveness on three occasions (post challenge on days 42, 70 and 84). There was no effect of treatment at any point on numbers of animals with live larvae present at 24 hours post challenge for any challenge occasion. There were significantly fewer animals with live larvae at 48 and 72 hours post challenge and L3 only at any point post challenge, for challenges on days 14, 42, 70 and 84. There were significantly lower numbers of live larvae at 24, 48 and 72 hours post challenge for challenges on days 14 (24 hours post challenge only), 21 (24 hours post challenge only), 42 (48 and 72 hours post challenge only), 70, 84 and 105 (48 and 72 hours post challenge only). There were significantly lower numbers of L3 present at 72 hours post challenge compare to the controls following challenge on days 14, 42, 70, 84 and 105.

Formulation B has significantly lower numbers of larvae (L1, L2 and L3) compared to Formulation A on days 22, 45, 85 and 86. Formulation A had significantly lower numbers of larvae (L1, L2 and L3) compared to Formulation B on day 106.

Overall all treatments demonstrated high levels of effectiveness against blowfly (*Lucilia* spp.) challenge in sheep. T2 was the least effective overall, and Formulation A (T2, T3 and T4) appeared to be less effective overall than Formulation B (T5, T6 and T7), with T5 having the greatest number of occasions of greater than 90% effectiveness and T6 and T7 have the highest effectiveness observed of 98.98% following challenge on day 84. All treatments demonstrated effectiveness to challenge on day 105 (15 weeks post treatment), demonstrating persistent efficacy, with three treatment groups having in excess of effectiveness, however, T7 had dropped to 62.21% effectiveness. There were no adverse events connected to treatment.

T3 had greater than 90% effectiveness on four challenge occasions, with 91.59%, 96.22%, 97.46% and 97.53% effectiveness following challenge on days 14, 42, 70 and 84 when comparing numbers of live L3 to the control group at any point post challenge.

T4 had greater than 90% effectiveness on four challenge occasions, with 91.59%, 97.46%, 98.53% and 98.48% effectiveness following challenge on days 14, 70, 84 and 105 when comparing numbers of live L3 to the control group at any point post challenge.

T5 had greater than 90% effectiveness on five challenge occasions, with 91.59%, 94.59%, 97.46%, 98.53% and 91.95% effectiveness following challenge on days 14, 42, 70, 84 and 105 when comparing numbers of live L3 to the control group at any point post challenge.

T6 had greater than 90% effectiveness on four challenge occasions, with 96.22%, 93.36%, 98.98% and 96.73% effectiveness following challenge on days 42, 70, 84 and 105 when comparing numbers of live L3 to the control group at any point post challenge.

T7 had greater than 90% effectiveness on four challenge occasions, with 96.22%, 97.46% and 98.98% effectiveness following challenge on days 42, 70 and 84 when comparing numbers of live L3 to the control group at any point post challenge.

The percentage effectiveness observed for each treatment group numbers of live larvae (L1, L2 and L3) against the controls at 48 and 72 hours post challenge were similar to those observed comparing numbers of live L3 at any point post challenge to the controls.

## Conclusion

Overall all treatments demonstrated high levels of effectiveness against blowfly (*Lucilia* spp.) challenge in sheep. T2 was the least effective overall, and Formulation A (T2, T3 and T4) appeared to be less effective overall than Formulation B (T5, T6 and T7), with T5 having the greatest number of occasions of greater than 90% effectiveness and T6 and T7 have the highest effectiveness observed of 98.98% following challenge on day 84. All treatments demonstrated effectiveness to challenge on day 105 (15 weeks post treatment), demonstrating persistent efficacy, with three treatment groups having in excess of 90% effectiveness, however T7 had dropped to 62.21% effectiveness. There were no adverse events connected to treatment.

## Residue Analysis

Samples of ovine liner, kidney, muscle, fast and fleece were chromatographically analysed for the enantiomers of Novaluron. The analysis were acceptable with reference to assay acceptance criteria.

## Claims

1. Novaluron for use in the prophylaxis of blow fly strike in sheep.

2. Novaluron for use as claimed in claim 1 wherein the Novaluron is in the form of a pour-on formulation.

3. Novaluron for use as claimed in claim 2 wherein the pour-on formulation comprises from 1% to 5% by weight of Novaluron, diethylene glycol butyl ether, and polyvinylpyrollidone.

4. Novaluron for use as claimed in claim 3 wherein Novaluron is present in the formulation at a concentration of from 2% to 4% by weight.

5. Novaluron for use as claimed in claim 3 or 4 wherein Novaluron is present in the formulation at a concentration of 3% by weight.

6. Novaluron for use as claimed in any of claims 3 to 5 wherein diethylene glycol butyl ether comprises from 90 to 95% w/w of the formulation.

7. Novaluron for use as claimed in any of claims 3 to 6 wherein polyvinylpyrollidone (PVP) comprises from 3 to 6% w/w of the formulation.

8. Novaluron for use as claimed in any of claims 3 to 7 wherein the PVP has a molecular weight of 5 to 500 kDa.

9. Novaluron for use as claimed in any of claims 3 to 8 wherein the PVP has a molecular weight of from 40 to 80 kDa.

**Patentansprüche**

1. Novaluron für die Verwendung bei der Prophylaxe von Schmeißfliegenbefall bei Schafen.

2. Novaluron für die Verwendung nach Anspruch 1, wobei das Novaluron in Form einer aufgießbaren Formulierung vorliegt.

3. Novaluron für die Verwendung nach Anspruch 2, wobei die aufgießbare Formulierung von 1 Gewichts-% bis 5 Gewichts-% Novaluron, Diethylenglycolbutylether und Polyvinylpyrolidon umfasst.

4. Novaluron für die Verwendung nach Anspruch 3, wobei Novaluron in der Formulierung in einer Konzentration von 2 Gewichts-% bis 4 Gewichts-% vorliegt.

5. Novaluron für die Verwendung nach Anspruch 3 oder 4, wobei Novaluron in der Formulierung in einer Konzentration von 3 Gewichts-% vorliegt.

6. Novaluron für die Verwendung nach einem der Ansprüche 3 bis 5, wobei Diethylenglycolbutylether von 90 bis 95 m/m-% der Formulierung umfasst.

7. Novaluron für die Verwendung nach einem der Ansprüche 3 bis 6, wobei Polyvinylpyrolidon (PVP) von 3 bis 6 m/m-% der Formulierung umfasst.

8. Novaluron für die Verwendung nach einem der Ansprüche 3 bis 7, wobei das PVP ein Molekulargewicht von 5 bis 500 kDa aufweist.

9. Novaluron für die Verwendung nach einem der Ansprüche 3 bis 8, wobei das PVP ein Molekulargewicht von 40 bis 80 kDa aufweist.

**Revendications**

1. Novaluron pour une utilisation dans la prophylaxie d'attaques par des calliphoridés chez le mouton.

2. Novaluron pour une utilisation selon la revendication 1, le novaluron se trouvant sous la forme d'une formulation par voie transcutanée.

3. Novaluron pour une utilisation selon la revendication 2, la formulation par voie transcutanée comprenant de 1% à 5% en poids de novaluron, du diéthylène glycol butyléther, et de la polyvinylpyrrolidone.

**4.** Novaluron pour une utilisation selon la revendication 3, le novaluron étant présent dans la formulation selon une concentration allant de 2% à 4% en poids.

**5.** Novaluron pour une utilisation selon la revendication 3 ou 4, le novaluron étant présent dans la formulation selon une concentration de 3% en poids.

**6.** Novaluron pour une utilisation selon l'une quelconque des revendications 3 à 5, le diéthylène glycol butyléther comprenant de 90 à 95% p/p de la formulation.

**7.** Novaluron pour une utilisation selon l'une quelconque des revendications 3 à 6, la polyvinylpyrrolidone (PVP) comprenant de 3 à 6% p/p de la formulation.

**8.** Novaluron pour une utilisation selon l'une quelconque des revendications 3 à 7, la PVP ayant un poids moléculaire allant de 5 à 500 kDa.

**9.** Novaluron pour une utilisation selon l'une quelconque des revendications 3 à 8, la PVP ayant un poids moléculaire allant de 40 à 80 kDa.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011120427 A

- US 5089525 A

**Non-patent literature cited in the description**

- *CHEMICAL ABSTRACTS,* 3087-16-9